# EUROPEAN PATENT APPLICATION

(11) **EP 2 180 009 A1**
(43) Date of publication of application: **28.04.2010**
(21) Application number: 09290718.7
(22) Date of filing: 18.09.2009
(51) Int. Cl.: C07K 14/755

(54) **B-domain-deleted recombinant human Factor VIII.**

(30) Priority: 18.09.2008 BR PI0805767
(71) Applicant: Fundacao Hemocentro de Ribeirao Preto, 14051-140 Ribeirao Preto - SP (BR); Universidade de São Paulo, 05508-900 São Paulo - SP (BR)
(72) Inventor: Fontes, Aparecida Maria, 14051-140 Ribeirao Preto - SP (BR); Covas, Dimas Tadeu, 14051-140 Ribeirao Preto - SP (BR)
(74) Representative: Novagraaf Technologies

(57) **Abstract**

The present invention refers to a recombinant human blood coagulation factor VIII protein and a composition containing it.

The present invention also refers to the use of the protein or composition of the invention for manufacturing a medicine for treating hemophilia A.

Additionally, the present invention refers to the method of obtaining a recombinant human blood coagulation factor VIII protein.

A further object of the present invention is a recombinant protein obtained by the method described herein, and its use in the preparation of a medicine for the treatment of hemophilia A.

## Description

### FIELD OF THE INVENTION

The present invention refers to a recombinant human blood coagulation factor VIII protein (FVIII), to a composition containing it and its uses in preparing a medicine for the treatment of hemophilia A.

Additionally, the present invention refers to the method of obtaining a recombinant human blood coagulation factor VIII protein (FVIII).

### BACKGROUND OF THE INVENTION

Hemophilia A is a recessive disease linked to gender, caused by the deficiency of factor VIII of blood coagulation in plasma.

Patients with hemophilia A may often present hematomas. Also common are hemarthroses (bleeding at the joints) at the ankles, knees, hips and elbows. They are often painful, and repeated episodes may lead to the destruction of the synovia and a decrease in articular function. Intracranial bleeding is also common, and can lead to death in hemophiliacs.

Factor VIII (FVIII) is a synthesized procofactor glycoprotein and is released into the blood stream by the vascular endothelium. In the circulating blood, it is mainly linked to the von Willebrand (vWF) factor forming a stable complex. In the coagulation cascade, the VIIIa factor associates with the factor IXa (factor Vllla:factor IXa), which converts factor X into factor Xa. The latter associates to the prothrombin complex (factor Va:factor Xa) which results in the conversion of the prothrombin into thrombin (IIa). The thrombin, in turn, displays procoagulant activities, converting the fibrinogen into fibrin, causing platelet activation and activating the factor XIII of the coagulation, which, in turn, stabilizes the fibrin coagulate.

FVIII products derived from human plasma were routinely used in the art for treating individuals with hemophilia A. Concentrates of FVIII were developed, and the use of these concentrates increased the duration and qualified of life of people with hemophilia A. Yet these products infected the patients with the hepatitis B (HBV) virus, the hepatitis C (HCV) virus and the human immunodeficiency (HIV) virus.

The drawbacks related to the concentrates derived from plasma, purified or not, stimulated attempts to develop recombinant FVIII products for use in patients with hemophilia A.

Therefore, at the beginning of the 1990s, pharmaceutical companies began developing products with the synthetic recombinant factor VIII (rFVIII).

The gene that encodes the FVIII protein is situated in region 28 of long arm of chromosome X (Xq28) and has a length of 186 Kb with a highly complex structure (Poustka A, Dietrich A, Langenstein G, Toniolo D, Warren ST and Lehrach H. "Physical map of human Xq27-qter: localizing the region of the fragile X mutation". Proc Natl Acad Sci USA 1991;88:8302-6). The RNA relating to the FVIII is comprised of 9,010 nucleotides, having a small 5' untranslated region (150 nucleotides), the region between the initiator codon and the terminator codon (7056 nucleotides), and a long 3' untranslated region (Wood WI, Capon DJ, Simonsen CC, Eaton DL, Gitschier J, Keyt B, Seeburg PH, Smith DH, Hollingshead P, Wion KL et al. "Expression of active human factor VIII from recombinant DNA clones". Nature 1984;312:330-7).

The protein deduced from the sequence of nucleotides of cDNA of FVIII contains 2351 amino acids, of which the first nineteen amino acids represent a peptide signal sequence, and the other 2331 amino acids the mature protein. This protein presents three domains called A (330 amino acids), B (980 amino acids) and C (160 amino acids), with three repetitions of domain A, two of domain C and a single domain B. This domain presents the following arrangement: A1-A2-B-A3-C1-C2, the heavy chain is constituted by domains A1-A2-B and the light chain by domains A3-C1-C2.

As a person skilled in the art knows, the larger the molecule to be produced, the lesser its yield, and the harder it will be to manipulate.

Therefore, the complexity and large size of the factor VIII led researchers to focus their initial studies on investigating the most suitable conditions (such as cell line and vector) for producing biologically active factor VIII.

The first plasmidial vectors that allowed the expression of recombinant FVIII in mammal cells present the basic characteristics of expression vectors, including a promoter region (promoter SV40 or adenovirus-2) and a polyadenylation signal (of the gene that encodes the surface antigen of the hepatitis virus). In these initial studies, the expression of complete rFVIII was cloned in cell lines of murines (COS and 62.2 - cell line derived from the kidney of a Chinese hamster), and the biological activity levels of factor VIII present in the supernatant of these cell cultures were in the order of 0.01 IU/ml (Wood WI, Capon DJ, Simonsen CC, Eaton DL, Gitschier J, Keyt B, Seeburg PH, Smith DH, Hollingshead P, Wion KL et al. "Expression of active human factor VIII from recombinant DNA clones". Nature 1984;312:330-7) and (Toole JJ, Knopf JL, Wozney JM, Sultzman LA, Buecker JL, Pittman DD, Kaufman RJ, Brown E, Shoemaker C, Orr EC et al. "Molecular cloning of a cDNA encoding human antihaemophilic factor". Nature 1984;312:342-7).

Next, Pavirani *et al.* (Pavirani A, Meulien P, Harrer H, Dott K, Mischler F, Wiesel ML, Mazurier C, Cazenave JP and Lecocq JP. "Two independent domains of factor VIII co-expressed using recombinant vaccinia viruses have procoagulant activity". Biochem Biophys Res Commun 1987;145:234-40) proposed two changes for the production of factor VIII: the use of viral vectors and the co-transfection of the heavy chain with the light chain, since it is known in the art that domain B is not necessary for the biological activity of factor VIII. This study analyzed the biological activity of the complete factor VIII or after co-infection of the heavy and light chains of factor VIII produced in cells BHK was in the order of 0.1 IU/mL or 0.01 IU/mL, respectively.

Subsequently, recombinant constructions were carried out containing cDNA relating to the factor VIII in four different viral vectors, including adenoviral, adeno-associated, retroviral and lentiviral vectors. In these studies, functional assays of the recombinant factor VIII present in different cell lines demonstrated varying biological activity levels, in accordance with the cell line and the vector type. For example, cell lines transfected with adeno-associated vectors showed biological activity levels in the order of 0.02-0.08 IU/mL (Chao H, Mao L, Bruce AT and Walsh CE. Sustained expression of human factor VIII in mice using a parvovirus-based vector. Blood 2000;95:1594-9), whereas lines transfected with rFVIII-carrying adenoviral vectors showed biological activity levels between 0.25-3.15 IU/mL (Andrews JL, Weaver L, Kaleko M and Connelly S. Efficient adenoviral vector transduction and expression of functional human factor VIII in cultured primary human hepatocytes. Haemophilia 1999;5:160-8). Furthermore, studies using primary cultures and retroviral vectors showed the generation of a rFVIII-carrying population with biological activity levels between 0.3-0.7 IU/mL. (Van Damme A, Chuah MK, Dell'accio F, De Bari C, Luyten F, Collen D and VandenDriessche T. Bone marrow mesenchymal cells for haemophilia A gene therapy using retroviral vectors with modified long-terminal repeats. Haemophilia 2003;9:94-103).

It is important to emphasize that the aforementioned vectors used in the studies carried out in the state of the art are monocistronic, that is, they permit the expression of a single mRNA from the promoting region, and do not present a selection marker gene. Hence, in these cases, the expression and activity of the rFVIII is investigated transitorily, between 24 and 72 hours after transfection.

Yet about 18 years ago, this situation changed with the discovery that the translation control of certain messenger RNAs could be governed by a mechanism independent of its "cap" structure. In this case, the translation of the messenger RNA would depend on a specific sequence inside the mRNA, recognized by the ribosomal RNA, called IRES (internal ribosome entry site). Among its different applications, this discovery led to the development of bicistronic plasmidial DNA vectors that permit the expression of two distinct genes: the selection marker gene and the gene of interest, from a single promoter region (Gurtu V, Yan G and Zhang G. IRES bicistronic expression vectors for efficient creation of stable mammalian cell lines. Biochem Biophys Res Commun 1996;229:295-8).

With the use of bicistronic vectors, there are three main strategies used to generate a cell population with stable expression of rFVIII, including: 1) selection of the transgenic population by flow cytometry by analyzing the expression of the GFP (Green Fluorescent Protein); 2) treatment of the transgenic population with geneticin by way of the co-expression of the gene that encodes for neomycin and 3) treatment of the transgenic population with methotrexate by way of the co-expression of the gene that encodes for dihydrofolate reductase (DHFR).

In the first case, fibroblast lines NIH3T3/rFVIIIAB+/GFP+ genetically modified with the retroviral system and selected with the GFP expression showed the secretion of the biologically active rFVIII with a level of 0.93±0.13 IU/106 cells (Moayeri M, Ramezani A, Morgan RA, Hawley TS and Hawley RG. "Sustained phenotypic correction of hemophilia in mice following oncoretroviral-mediated expression of a bioengineered human factor VIII gene in long-term hematopoietic repopulating cells". Mol Ther 2004;10:892-902). Further studies showed biological activity levels between 1.0-5.0 UI/mL, in human hepatic cell lines when transduced with lentiral vectors carrying the cytomegalovirus (CMV) promoter (Picanco V, Heinz S, Bott D, Behrmann M, Covas DT, Seifried E and Tonn T. "Recombinant expression of coagulation factor VIII in hepatic and non-hepatic cell lines stably transduced with third generation lentiviral vectors comprising the minimal factor VIII promoter". Cytotherapy 2007;9:785-94).

Other research groups have adopted the treatment with geneticin to select a cell population with stable expression of rFVIII. In this case, lines of Cos-7 cells (monkey kidney) or SMMC-7721 (human hepatoma) were transfected with rFVIII, using the plasmidial system. After the selection with geneticin, a secretion of rFVIII was diagnosed with a biological activity of 0.24±0.005 IU/10⁶ cells and 0.74±0.003 IU/10⁶ cells, respectively (Chen C, Fang XD, Zhu J, Wu XF, Zhang ZC, Gu JX, Wang ZY and Chi CW. "The gene expression of coagulation factor VIII in mammalian cell lines". Thromb Res 1999;95:105-15).

A selection with methotrexate is another widely used form of treatment by virtue of its effectiveness and low cost. In this sense, CHO cell lines deficient in dihydrofolate reductase (DHFR) were transfected with rFVIII carrying bicistronic plasmides, and the neomycin gene. After transfection, double selection was carried out, initially with geneticin, followed by treatment with methotrexate. The analysis of the biological activity using the activated partial thrombopastin time test showed the generation of a cellular clone with a production of rFVIII between 0.5-2 IU/10⁶ cells (Chun BH, Park SY, Chung N and Bang WG. "Enhanced production of recombinant B-domain deleted factor VIII from Chinese hamster ovary cells by propionic and butyric acids". Biotechnol Lett 2003;25:315-9).

Therefore, the advent of bicistronic vectors permitted the stable expression of recombinant FVIII in different cell lines of mammals, and different strategies for selecting a cell clone carrying high levels of rFVIII have been developed in the state of the art.

The recombinant blood coagulation factor VIII constitutes a highly safe medicine for the treatment of individuals with Hemophilia A. In some countries such as Canada and Ireland, it is the only source of treatment for these patients, and has been used not only as therapy, but also as prophylaxis. In other countries, such as the USA, 70% of hemophiliacs are treated with the recombinant form of this protein, whereas the other 30% depend on factor VIII from the plasma of healthy individuals.

Examples of recombinant products used in the art include: KOGENATE and KOGENATE FS by Bayer, RECOMBINATE by Baxter, and REFACTO by Wyeth/Genetics Institut. In these cases, the recombinant protein is produced in CHO cells (Chinese hamster ovary) or in BHK (newborn hamster kidney), which are stably transfected as complete FVIII or without domain B (Lee CA, Owens D, Bray G, Giangrande P, Collins P, Hay C, Gomperts E, Schroth P and Barrowcliffe T. "Pharmacokinetics of recombinant factor VIII (recombinate) using one-stage clotting and chromogenic factor VIII assay". Thromb Haemost 1999;82:1644-7).

Hemophilia A affects 1 in every 5,000 male births worldwide. In Brazil, there are an estimated 9,000 registered hemophiliacs who under ideal conditions would require 630 million UI of factor VIII for their adequate treatment (70,000 UI/patient/year).

As the recombinant products have been produced in just some regions of the world, and by few companies, there is a shortage in the supply of the product.

Additionally, it was observed that the recombinant factor VIII produced by non-human lines, may cause the patient to develop activity-neutralizing antibodies (inhibitors).

Therefore, the high cost of recombinant factor VIII, its limited quantity available on the market and the possibility of developing inhibitor antibodies, are factors that have encouraged researchers to develop new formulations of rFVIII, making it more accessible to the population of hemophiliacs A, and clinically more effective and feasible.

### BRIEF DESCRIPTION OF THE INVENTION

Based on an in-depth study of the molecular mechanisms that govern the expression of the gene that encodes the FVIII protein, as well as a detailed biochemical characterization of the FVIII protein, the Filing Applicant has managed to develop a new recombinant FVIII molecule, produced at high levels, particularly in human cell lines.

The molecule of the present invention, besides being produced at high levels, presents increased biological activity and does not present the drawback of inducing the developing of inhibitor antibodies, since it is produced from human cell lines.

Therefore, the present invention refers to a recombinant human blood coagulation factor VIII protein which presents reduced domain B, wherein 17 to 19 amino acids are preserved from the domain B of natural FVIII, wherein 6 to 8 amino acids are preserved from the N-terminal, and 11 to 13 amino acids are preserved from C-terminal of the original domain B, and which presents a serine and a threonine among the amino acids conserved from the N-terminal and C-terminal.

Another object of the present invention is a composition the comprises the recombinant factor VIII protein of the present invention.

The present invention also refers to the use of the recombinant protein described herein, or the composition containing it, to manufacture a medicine for treating hemophilia A.

An additional object of the present invention is the method of obtaining a recombinant human blood coagulation factor VIII protein wherein the method comprises:
a) obtaining a DNA molecule that encodes the human blood coagulation factor VIII;
b) amplification of the molecule of step (a) by PCR, using primers that are specific for the sequence of nucleotides that encodes domain B, wherein the 3' primer is specific for a sequence that encodes 6 to 8 amino acids of the N-terminal of the original domain B, and the 5' primer is specific for a sequence that encodes 11 to 13 amino acids of the C-terminal of the original domain B, wherein one of the primers also presents nucleotides that encode serine (S), and the other primer presents nucleotides that encode threonine (T), which are inserted between the sequences of nucleotides conserved from the N-terminal and C-terminal of the domain B;
c) introduction of the recombinant DNA FVIII molecule obtained by step (b) into a vector;
d) introduction of the IRES element into the vector of step (c), by way of restriction enzymes, after isolation by PCR;
e) transfection of human cells with the vector obtained by step (d);
f) treatment of the culture of human cells with increasing concentrations of chemotherapeutic drugs and stringency;
g) cultivation of recovered cultures; and
h) recovery of the recombinant factor VIII obtained by said cultures.

Additionally, the object of the present invention is the recombinant human blood coagulation factor VIII protein obtained by the method above, and the use of this protein to manufacture a medicine for the treatment of hemophilia A.

### BRIEF DESCRIPTION OF THE DRAWINGS

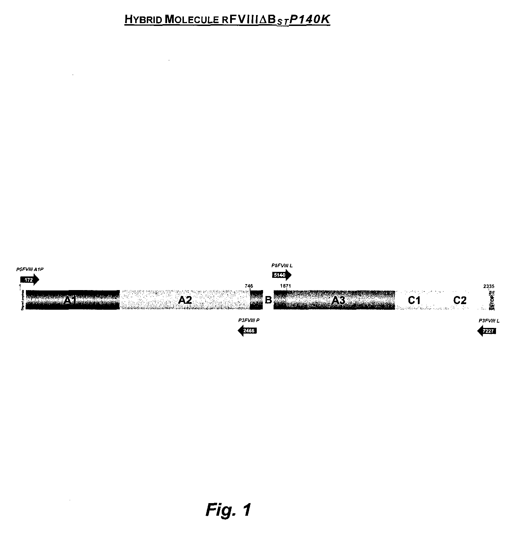

Figure 1 represents a schematic figure of the hybrid molecule of the present invention (heavy chain + light chain + 17 amino acids of domain B + 2 fusion amino acids). Said molecule is a single molecule, in which the fusion of the heavy chain (domains A1 and A2) was carried out with the light chain (domains A3, C1 and C2). In the union of the two chains, 17 amino acids were conserved from domain B, plus 2 extra amino acids, among the said 17 amino acids.

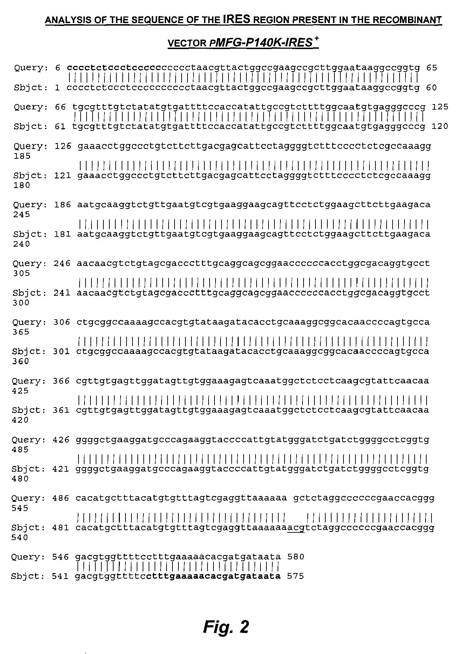

Figure 2 represents an analysis of the sequence of the IRES region present in the vector of the present invention. The sequence in bold type was inserted into all the clones owing to the primer used. The boldface bases demonstrate the adenine base which was added, and the inversion of GC for CG.

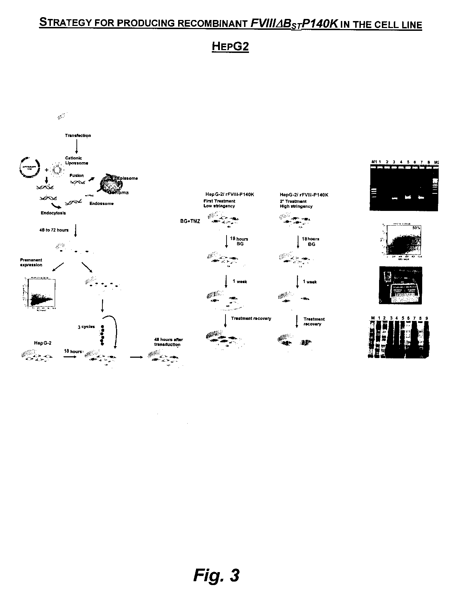

Figure 3 illustrates the strategy for producing recombinant FVIII of the present invention in the cell line HepG2.

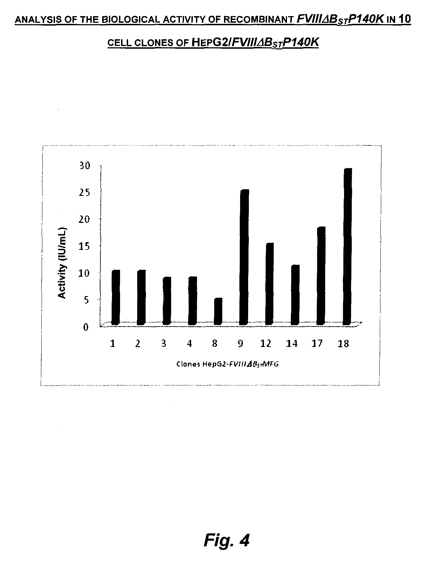

Figure 4 illustrates an analysis of the biological activity of recombinant FVIII of the present invention in 10 cell clones of HepG2 containing said molecule of the invention. Clone 18 showed higher levels of FVIII in the order of 29 IU/mL as evaluated by the activated partial prothrombin test.

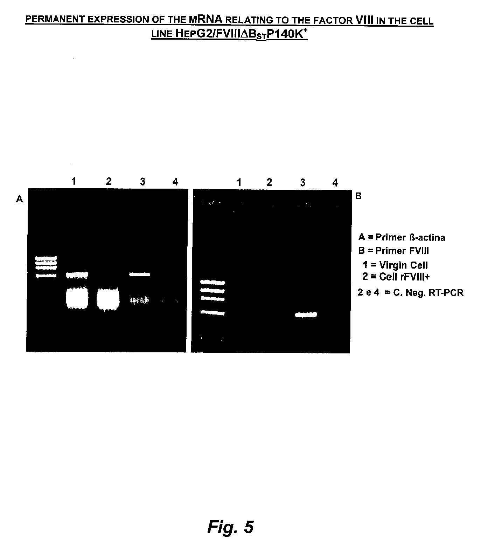

Figure 5 illustrates the permanent expression of the mRNA relating to the factor VIII of the invention in the cell line HepG2, by way of agarose gel with ethidium bromide staining after electrophoresis of the RT-PCR products. About 2 µg of total RNA was submitted to reaction with the reverse transcriptase, using specific primers of FVIII (3B), and β-actin (1A and 3A). Next, 1/10 of the RT reaction product is submitted to PCR reaction using specific primers of FVIII (bands 1 and 3 -B), and β-actin (bands 1 and 3 -A). As bands 1 and 2 present the RNA of cells HepG2 not transduced, showing in 1 a band of 612 pb relating to the β-actin gene, and in the other amplification absence controls. Bands 3 and 4 present the RNA of the cells HepG2 transduced with FVIII of the invention, showing the fragments of 612pb and 546pb, relating to the β-actin gene and FVIII, respectively. The other bands (2 and 4) represent the negative control of the RT reaction, in which reverse transcriptase was not added. The first bands show the DNA molecular weight marker φX 174 (fragments 1353; 1078; 872; 603 and 310 pb).

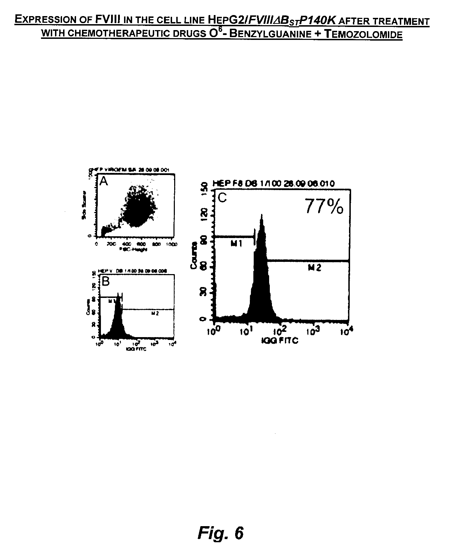

Figure 6 illustrates the expression of FVIII of the present invention in the cell line HepG2, after treatment with chemotherapeutic drugs O⁶-Benzylguanine + Temozolomide. (A) dot plot SSCXFSC graph, showing the size and cellular complexity of virgin HepG2; (B) bar graph showing the absence of the expression of FVIII in virgin HepG2 cells; and (C) bar graph showing the expression level of FVIII by way of the specific link with the anti-antibody in the HepG2 cellular population.

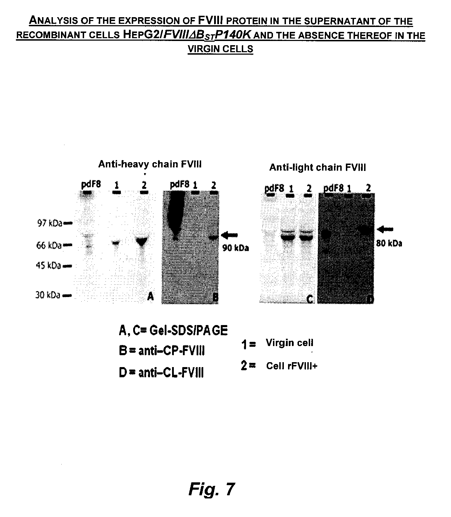

Figure 7 illustrates an analysis of the expression of FVIII protein of the present invention in the supernatant of the recombinant cells HepG2, and the absence thereof in the virgin cells. To the left, the antibody anti-heavy chain was used and to the right, the antibody anti-light chain. A and C represent electrophoresis in Phast System with gel SDS/PAGE 12.5%, with Comassie blue R250 0.25% staining. Band pdF8 represents the lyophilized concentrate of commercial human FVIII, band 1 represents the supernatant of virgin cell HepG2, and band 2 represents the supernatant of cell recombinant HepG2 with the FVIII of the invention. B and D represent immunodetection of the protein transferred to the PVDF membrane by "Western blotting". It is possible to observe the immunoreactive band of the expected size of 90 kDa in the incubated membrane with the antibody anti-heavy chain of FVIII, and an immunoreactive band of the expected size of 80 kDa in the incubated membrane with the antibody anti-light chain of FVIII. The band on the left shows the molecular weight marker (bands 97, 66, 45 and 30 kDa).

### DETAILED DESCRIPTION OF THE INVENTION

In a first embodiment, the present invention refers to the recombinant human blood coagulation factor VIII protein, which comprises the reduced domain B, wherein 17 to 19 amino acids are preserved from domain B of natural FVIII, wherein 6 to 8 amino acids are preserved from N-terminal, and 11 to 13 amino acids are preserved from C-terminal of the original domain B, and which presents a serine and a threonine among the amino acids conserved from the N-terminal and C-terminal, and the recombinant protein is preferably produced by human lines.

In a particular embodiment, the human cell lines are selected from among hepatic lines. Preferably, the human line used by the present invention to obtain the recombinant human blood coagulation factor VIII protein is HepG2.

The recombinant human blood coagulation factor VIII protein of the present invention is produced in high levels, since it has a smaller structure than natural FVIII, and presents increased biological activity, being suitable for use in the treatment of hemophilia A.

The present invention also refers to the composition that comprises the recombinant factor VIII protein of the present invention and pharmaceutically acceptable vehicles, excipients or stabilizers.

The composition according to the present invention can be liquid, semi solid or solid, and can be adapted for any enteral or parenteral administration route, either immediate or modified release. In a particular embodiment, said composition is adapted for oral administration, more particularly, in the form of pills, capsules, dyes, emulsions, liposomes, microcapsules or nanoparticles.

Vehicles, excipients or stabilizers suitable for the invention are, for example, and without any limitation, those cited in the work Remington's Pharmaceutical Sciences, by US publishing house Mack Publishing, or also in the European Pharmacopoeia or Brazilian Pharmacopoeia.

In another aspect, the present invention refers to the use of the recombinant FVIII protein of the invention, or compositions containing it, for the preparation of medicines for treating hemophilia A.

In another embodiment, the present invention refers to a method of obtaining a recombinant human blood coagulation factor VIII protein, wherein the method comprises:
a) obtaining a DNA molecule that encodes the human blood coagulation factor VIII;
b) amplification of the molecule of step (a) by PCR, using primers that are specific for the sequence of nucleotides that encodes domain B, wherein the 3' primer is specific for a sequence that encodes 6 to 8 preserved amino acids are from N-terminal, and 11 to 13 preserved amino acids are from the C-terminal of the original domain B, wherein one of the primers also presents nucleotides that encode serine (S), and the other primer presents nucleotides that encode threonine (T), which are inserted between the sequences of nucleotides conserved from N-terminal and C-terminal of domain B;
c) introduction of the recombinant DNA FVIII molecule obtained by step (b) into a vector;
d) introduction of the IRES element into the vector of step (c), by way of restriction enzymes;
e) transfection of human cells with the vector obtained by step (d);
f) treatment of the culture of human cells with increasing concentrations of chemotherapeutic drugs and stringency;
g) cultivation of recovered cultures; and
h) recovery of the recombinant factor VIII obtained by said cultures.

According to a preferred embodiment, the primers used in step (b) present the following sequence (1 and 2 being for the N-terminal region and 3 and 4 for the C-terminal region):
Primer 1 - 5'-TTCTATCACACGTGACCATGCAAATAGAGCTCTCCACC-3'
Primer 2 - 5'-TTCTATAAAGTACTTGAATTCTGGGAGAAGCTTCTTG-3'
Primer 3 - 5'-TTCTATAAAGTACTCAAAACCCACCAGTCTTGAAAC-3'
Primer 4 - 5'-TTCTATACACACGTGTCAGTAGAGGTCCTGTGCC TC-3'

The conditions used for the PCR reaction are known by persons skilled in the art and are described in the state of the art.

Step (c) is particularly realized starting from the digestion of the molecule of step (b) with a specific endonucleases, preferably Pme *I,* and link of the molecule thus treated to the DNA of the vector linearized with the same restriction enzymes.

Further, step (d) of the above method is carried out from the digestion of the vector of step (c) with specific endonucleases, preferably Pme *I* and *Nco I*, and link of the vector thus treated to the IRES linearized with the same restriction enzymes.

In a preferred embodiment, the vector used by the present invention is a retroviral vector, particularly, plasmidial retroviral. According to a preferred embodiment, the vector used is pMGF-P140K.

According to another aspect of the present invention, the transfection of human cells with the vector obtained by step (d) can be carried out by any method known in the art. Particularly, transfection is carried out by way of lipofectamine.

Particularly, the method of the present invention presents a retrovirus production system. Firstly, the FVIII is transfected in amphotropic retrovirus producing cells. Next, the human cell is then transduced with the retrovirus produced by the amphotropic line.

Moreover, the chemotherapeutic drugs used in the present invention for the treatment of the cultures transfected can be any one suitable from the state of the art, provided that the vector used contains a resistance gene thereto. Particularly, the chemotherapeutic drugs used are O⁶-Benzylguanine and Temozolomide, without excluding any other suitable selection system.

As described above, the selection of transduced cultures is performed by increasing treatment with chemotherapeutic drugs, with increasing stringency also. According to the present invention, the doses of chemotherapeutic drugs used in the first selection (low stringency) are comprised between 200 and 400 µg/mL, and the doses of chemotherapeutic drugs used in the second selection (high stringency) are comprised between 500 and 800 µg/mL.

The cultivation of cells is carried out under standard conditions, and can be determined by a person skilled in the art depending on the type of cell line used. Particularly, cultivation is carried out at a temperature of 37°C and 5% of CO2.

Further, the recovery of the recombinant factor VIII obtained by the method of the present invention can be carried out by any method known in the art.

The method of the present invention allows recombinant human FVIII proteins to be obtained in high levels. The proteins present high biological activity and do not have the drawbacks of those recombinant proteins in the art.

According to an additional embodiment, the present invention refers to the recombinant factor VIII protein obtained by the method described above, presenting reduced domain B, wherein 17 to 19 amino acids are preserved form domain B of natural FVIII, wherein 6 to 8 amino acids are preserved from N-terminal, and 11 to 13 amino acids are preserved from C-terminal of the original domain B, and which presents a serine and a threonine among the amino acids conserved from the N-terminal and C-terminal.

Yet another object of the present invention refers to the use of the protein obtained by the method of the present invention in the preparation of a medicine for treating hemophilia A.

The present invention may be understood in a clearer and more precise way by reading the examples below, which illustrate the present invention without presenting any limitative character.

### EXAMPLES

### EXAMPLE 1

### PRODUCTION OF MUTANT FVIII FROM HUMAN LINES

The human cell line HepG2 was transduced with the vector retroviral *FVII*Δ*B_{ST}P140K* that contains the recombinant FVIII of the present invention and the IRES element illustrated in figure 2. After the treatment with increasing concentrations of the chemotherapeutic drugs O⁶-Benzylguanine + Temozolomide for the selection of a cell population with high levels of rFVIII expression, cell cloning was carried out. Figure 3 shows a strategy used to generate the human cell line with stable expression of factor VIII of human blood coagulation, and high levels thereof.

The analysis of the biological activity of FVIII present in the supernatant of these cell clones showed a variation in the biological activity between 4.8 and 29 times higher than the factor VIII present in the blood plasma and than the recombinant factor VIII produced by murine lines described in the state of the art (figure 4).

### EXAMPLE 2

### EVALUATION OF THE PRODUCTION LEVEL OF THE RECOMBINANT MOLECULE OF THE INVENTION BY HUMAN CELL LINES HEPG2

The evaluation of the production level of the recombinant molecule *FVIII*Δ*B_{ST}P140K* by the human cell line HepG2 can be carried out by conventional RT-PCR; flow cytometry, activated partial thrombopastin time and *western blot.*

### CONVENTIONAL RT-PCR

The evaluation by conventional RT-PCR, illustrated in figure 5, enables a diagnosis of the presence of mRNA relating to coagulation factor VIII of the present invention using the following procedure: total RNA is extracted using the kit *RNAsy Mini kit* (Quiagen), according to the manufacturer's instructions. Next, 1 to 3 µg of total RNA is converted into cDNA, using the *Superscript II* (*Invitrogen*) kit, in accordance with the manufacturer's instructions, and 50 pmoles of random primer. Subsequently, the DNA fragment relating to factor VIII of the invention is amplified in a reaction mixture that contains: 2 µL of cDNA, 0.2 M of dNTPs; 1 U of the enzyme Taq DNA polimerase (*Amershan Bioscience*), 2.5 µL of buffer 10x of the respective enzyme (*Amershan Bioscience*) and 10 pmoles of each oligonucleotid P5FVIII5seq and P3FVIIIseq. This reaction was performed in a thermocycler with the program: 95°C for 2min; 35 cycles of 95°C for 40s, 56°C for 40s and 72°C for 1min; 72°C for 10min.

### FLOW CYTOMETRY

For intracellular marking of the FVIII recombinant protein of the present invention, about 1x10⁶ of HepG2/*FVII*Δ*B_{ST}P140K* cells are fixed with paraformaldehyde 1% (m/v) for 20 minutes at 4°C and, next, permeabilized with Tween 0.5% for 15 minutes at 37°C. After the washing step with PBS-BSA 2%, the cells are incubated with blocking solution (goat serum 10%) for 1 hour at 37°C. Thereafter, the diluted primary monoclonal antibody (QED) is placed in the solution PBS-BSA 2% (1:100), for approximately 24 hours at ambient temperature. Next, a wash is carried out with PBS-BSA 2%, and the cells are incubated with the secondary antibody diluted in solution PBS-BSA 2% (1:400), for 45 minutes at ambient temperature. After this period, a second wash is performed with PBS 1X, and the cells are eluted in 100µL of PBS 1X, for subsequent analysis of fluorescence emission by the recombinant cells by flow cytometry. The quantification of the fluorescence emission was performed with a cell suspension using 10,000 events (cells) in a laminar flow cytometer (*FACSort, Beckton Dickinson, San Jose, CA, USA*).

Evaluation by flow cytometry showed that the recombinant HepG2 cell population presents a stable expression of FVIII in the order of 77%, as illustrated in figure 6.

### ACTIVATED PARTIAL THROMBOPASTIN TIME (APTT) TEST

The evaluation of the biological activity is performed using the activated partial thrombopastin time test illustrated in table 1.

**TABLE 1**

| **HepG2-*FVIII***Δ***B_{ST}P140K* Clones** | **Biological Activity (IU/mL)** |
|---|---|
| 1 | 10.0 |
| 2 | 10.0 |
| 3 | 8.7 |
| 4 | 8.8 |
| 8 | 4.8 |
| 9 | 25.0 |
| 12 | 15.0 |
| 14 | 11.0 |
| 17 | 18.0 |
| 18 | 29.0 |

The APTT coagulation test measures the speed *in vitro* that a sample of plasma containing the FVIII takes to coagulate a sample of plasma deficient in FVIII. The parameter measured is the time required for prothrombin activity. Accordingly, the time taken for coagulation varies according to the concentration of FVIII present in the sample to be analyzed. In the present invention, the sample to be tested was diluted 1:10 in buffer (Owren's Veronal - Biomérieux) and, after 20 seconds, mixed with the plasma deficient in human FVIII (*Biomérieux, Durham*), phospholipids and a contact activator (*Platelin*® *LS - Biomérieux*). After incubation for approximately 240 seconds at 37°C, 100 µL of calcium chloride (CaCl₂ 0.25M) was added, and the time to form the coagulate was marked using the automatic coagulometer (*COAG-A-MATE*® *XM - Organon Teknika*), according to the manufacturer's instructions. Before starting the dosage, a standard calibration curve was built using FVIII plasma derivative (*Verify - Reference plasma - (Organon Teknika, Durham)*), reconstituted with 1mL of distilled water diluted for concentration of (1 U mL FVIII). Sic dilutions (1:5; 1:10; 1:20; 1:40; 1:80; 1:160) are carried out in a buffer (*Owren's Veronal - Biomerieux*), and used for building a curve for each sample. A linear relationship was drawn on a graph, with FVIII activity in logarithmic scale (abscissa) and the activated partial thrombopastin time (seconds), and the percentage of FVIII activity calculated was obtained with the average slant of the curve.

Table 1 shows the analysis of the biological activity of FVIII present in the supernatant of 10 cell clones of the HepG2/ *FVIII*Δ*B_{ST}P140K* cell population using the APTT test. It can be noted that the recombinant cell clones carrying FVIII after treatment with O⁶-Benzylguanine + Temozolomide, present biological activity levels of FVIII in the order of 4.8 to 29 times the level of factor VIII present in blood plasma.

### WESTERN BLOT

The characterization of the protein *FVIII*Δ*B_{ST}P140K,* produced by the cell line *HepG2*/*FVIII*Δ*B_{ST}P140K,* was made by *western blot*.

To prepare the gel, 7 mL of supernatant is collected from 1x10⁷ cells *HepG2*/ *FVIII*Δ*B_{ST}P140K* and submitted to concentration in columns *Centricon*® *Amicon*® *(Millipore)*, by centrifugation at 7500 rpm for 2 to 4 hours. Next, a wash is carried out using 3 ml of autoclaved H₂O *MilliQ*, by centrifugation at 6000 rpm. Subsequently, the columns were inverted for elution of the samples in approximately 150 µl of autoclaved H₂O *MilliQ*, and centrifuged at 6000 rpm for 15 minutes. After elution of the protein, the samples are quantified in a spectrophotometer using the Bradford Method.

To carry out the *Western Blot*, the samples were submitted to electrophoresis using the *Phast System*. About 10 µL of the sample (30 µg) was mixed with 10 µl of the sample buffer (10 mM Tris-HCl, pH 8.0; 2.5% SDS (m/v); 1 mM EDTA; 0.01% (v/v) of blue bromophenol), and 1.5 µl of β-mercaptoethanol (Sigma). The mixture is submitted to heating and applied to the gel. The electrophoresis is performed in a running buffer for the *PhastGel* (*SDS Buffer - Amersham Biociences*) at 100 V, for approximately 40 minutes. Two gels, exactly identical, are submitted to electrophoresis, one to be stained and the other for transfer. After the electrophoresis, the gel to be stained is incubated for 1 hour in fixing solution (ethanol 40% (v/v), acetic acid 10% (v/v), sufficient quantity of sterile water for 0.125L) and, subsequently, stained with Coomassie brilliant blue solution (1.3% phosphoric acid (v/v), 0.75 M (NH₄)₂ SO₄, 0.1% Coomassie B-Blue G250 (v/v)), for 18 hours. Next, the gel is washed 3 to 5 times with water MilliQ, and mounted between two sheets of cellophane paper for thorough drying.

After the electrophoresis, the proteins contained in the polyacrylamide gel are transferred to a nitrocellulose membrane, by the semi-dry electro-transfer method, with the use of the *Phast System - Amersham Biociences* apparatus, in accordance with the manufacturer's instructions. Electrophoresis is performed in a transfer buffer (0.25 M of Tris-Base; 0.96 M of glycine; 0.5% SDS (sodium duodecyl sulfate); sufficient quantity of sterile water for 1 L) at 90 V, for approximately 2 hours and 30 minutes. Once the transfer is concluded, the membrane is prepared for immunodetection.

The nitrocellulose membrane is submitted to blockage of unspecific sites by adding blocking solution TBS-T (10 mM Tris pH 7.5, 150 mM NaCl, 0.1% Tween-20), in 5% of powdered milk (m/v), for about 16 hours. After blocking, the membrane is washed twice quickly with TBS 1X, 0.1% Tween-20, and incubated with monoclonal primary antibody (QED), diluted in solution TBS-T (1:800) for 2 hours at ambient temperature, and under constant agitation. Next, the membrane is washed with TBS 1X, 0.1% Tween-20 (2X / 15 minutes; 3X / 5 minutes), and the membrane is incubated with the secondary antibody diluted in solution TBS-T (1:3000), for 1 hour. After incubation with the second antibody, the membrane is washed again and so begins the detection procedure. The *"ECL Western blotting"* (*Amersham Biosciences*) kit is used for detection in accordance with the manufacturer's instructions. Lastly, the membrane is exposed to the X-ray film for 15 seconds and processing is by KODAK automatic cameras.

As illustrated in figure 7, it is possible to observe a more intense immuno-reactive band of the expected size of 80 kDa, relating to the light chain of FVIII and of 90 kDa, relating to the heavy chain of FVIII, present in the recombinant HepG2/*FVIII*Δ*B_{ST}P140K* cells and the absence thereof in the virgin HepG2 cells.

As persons skilled in the art will fully appreciate, numerous changes and variations of the present invention are possible in light of the teachings above, without exceeding the scope of protection, as delimited by the claims appended hereto.

## Claims

1. RECOMBINANT HUMAN BLOOD COAGULATION PROTEIN OF FATOR VIII, **characterized by** comprising the reduced domain B, wherein 17 to 19 amino acids are preserved from domain B of natural FVIII, wherein 6 to 8 amino acids are preserved from N-terminal, and from 11 to 13 amino acids are preserved from C-terminal of original domain B, and which presents a serine and a threonine among the amino acids conserved from the N-terminal and C-terminal.

2. PROTEIN according to claim 1, **characterized** wherein 17 amino acids are preserved from domain B of natural FVIII, wherin 6 amino acids are preserved from N-terminal and 11 amino acids are preserved from C-terminal of the original domain B.

3. PROTEIN according to claim 1, **characterized by** being obtained from human cell lines.

4. PROTEIN according to claim 3, **characterized** wherein the cell lines are human hepatic.

5. PROTEIN according to claim 4, **characterized** wherein the human cell line is HepG2.

6. COMPOSITION, **characterized by** comprising a recombinant factor VIII protein, as described in any of claims 1 to 5, and pharmaceutically acceptable vehicles, excipients or stabilizers.

7. USE OF A RECOMBINANT FACTOR VIII PROTEIN, as described in any of claims 1 to 5, **characterized by** being in the preparation of a medicine for treating hemophilia A.

8. USE OF A RECOMBINANT FACTOR VIII PROTEIN, as described in any of claims 1 to 5, **characterized by** being for the treatment of hemophilia A.

9. USE OF A COMPOSITION, as described in claim 6,
**characterized by** being in the preparation of a medicine for treating hemophilia A.

10. USE OF A COMPOSITION, as described in claim 6, **characterized by** being for the treatment of hemophilia A.

11. METHOD OF OBTAINING A RECOMBINANT HUMAN BLOOD COAGULATION PROTEIN OF FATOR VIII, **characterized by** comprising:
a) obtaining a DNA molecule that encodes the human blood coagulation factor VIII;
b) amplification of the molecule of step (a) by PCR, using primers that are specific for the sequence of nucleotides that encodes domain B, where 3' primer is specific for a sequence that encodes 6 to 8 amino acids of the N-terminal of the original domain B, and 5' primer is specific for a sequence that encodes 11 to 13 amino acids of the C-terminal of the original domain B, wherein one of the primers also presents nucleotides that encode serine (S), and the other primer presents nucleotides that encode threonine (T), which are inserted between the sequences of nucleotides conserved from N-terminal and C-terminal of domain B;
c) introduction of the recombinant DNA FVIII molecule obtained by step (b) into a vector;
d) introduction of the IRES element into the vector of step (c), by way of restriction enzymes, after isolation by PCR;
e) transfection of human cells with the vector obtained by step (d);
f) treatment of the human cell culture with increasing concentrations of chemotherapeutic drugs and stringency;
g) cultivation of recovered cultures; and
h) recovery of the recombinant factor VIII obtained by said cultures.

12. METHOD according to claim 11, **characterized** wherein the primers used in step (b) present the following sequences:
Primer 1 - 5'-TTCTATCACACGTGACCATGCAAATAGAGCTCTCCACC-3'
Primer 2 - 5'-TTCTATAAAGTACTTGAATTCTGGGAGAAGCTTCTTG-3'
Primer 3 - 5'-TTCTATAAAGTACTCAAAACCCACCAGTCTTGAAAC-3'
Primer 4 - 5'-TTCTATACACACGTGTCAGTAGAGGTCCTGTGCC TC-3'.

13. METHOD according to claim 11, **characterized** wherein step (c) is carried out from the digestion of the molecule of step (b) with restriction enzymes, and link of the molecule thus treated to the DNA of the vector linearized with the same restriction enzymes.

14. METHOD according to claim 13, **characterized** wherein the restriction enzyme is *Pme I*.

15. METHOD according to claim 11, **characterized** wherein step (d) is carried out from the digestion of the vector of step (c) with restriction enzymes, and link of the vector thus treated to the IRES linearized with the same restriction enzymes.

16. METHOD according to claim 15, **characterized** wherein the restriction enzymes are *Pme I* and *Nco HI.*

17. METHOD according to claim 11, **characterized** wherein the vector is retroviral.

18. METHOD according to claim 17, **characterized** wherein the vector is pMGF-P140K.

19. METHOD according to claim 11, **characterized** wherein the transfection of the human cells with the vector obtained by step (d) is carried out by lipofectamine.

20. METHOD according to claim 11, **characterized by** presenting a retrovirus production system, wherein the FVIII is transfected into amphotropic retrovirus-producing cells.

21. METHOD according to claim 11, **characterized** wherein the chemotherapeutic drugs used in step (f) are O⁶-Benzylguanine and Temozolomide.

22. METHOD according to claim 11, **characterized** wherein the increasing doses of chemotherapeutic drugs of step (f) are comprised between 200 and 400 µg/ml in the first selection (low stringency) and between 500 and 800 µg/ml in the second selection (high stringency).

23. RECOMBINANT HUMAN BLOOD COAGULATION FACTOR VIII PROTEIN, **characterized by** being obtained by way of the method as described in any of claims 11 to 22, presenting reduced domain B, wherein 17 to 19 amino acids are preserved from domain B of natural FVIII, wherein 6 to 8 amino acids are preserved from the N-terminal, and 11 to 13 amino acids are preserved from the C-terminal of the original domain B, and that presents a serine and a threonine among the amino acids conserved from the N-terminal and the C-terminal.

24. PROTEIN according to claim 23, **characterized** wherein 17 amino acids are preserved from domain B of natural FVIII, where 6 amino acids are preserved from the N-terminal and 11 amino acids are preserved from the C-terminal of the original domain B.

25. USE OF A RECOMBINANT FACTOR VIII PROTEIN, as described in either of claims 23 or 24, **characterized by** being for the preparation of a medicine for treating hemophilia A.
